# EUROPEAN PATENT APPLICATION

(11) **EP 3 427 683 A1**
(43) Date of publication of application: **16.01.2019**
(21) Application number: 17180744.9
(22) Date of filing: 11.07.2017
(51) Int. Cl.: A61B 18/20, A61B 18/00

(54) **A CUTTING ASSEMBLY FOR A HAIR CUTTING DEVICE**

(71) Applicant: Koninklijke Philips N.V., 5656 AE Eindhoven (NL)
(72) Inventor: JURNA, Martin, 5656 AE Eindhoven (NL); THUMMA, Kiran Kumar, 5656 AE Eindhoven (NL); BOAMFA, Marius Iosif, 5656 AE Eindhoven (NL); JOHNSON, Mark Thomas, 5656 AE Eindhoven (NL)
(74) Representative: de Haan, Poul Erik

(57) **Abstract**

There is provided a cutting assembly for use in a hair cutting device. The cutting assembly comprises an optical waveguide having a light receiving end and a sidewall, wherein a portion of the sidewall forms a cutting face for contacting hair. The cutting assembly further comprises a housing element to support the optical waveguide. The cutting assembly further comprises a biasing member acting upon the optical waveguide such that, upon the optical waveguide becoming severed, thereby creating two severed faces of the optical waveguide, the severed face attached to the light receiving end of the optical waveguide is caused to face the housing element. A hair cutting device is also disclosed.

## Description

### FIELD OF THE INVENTION

The invention relates to a cutting assembly for use in a hair cutting device and, more particularly, to a cutting assembly having an optical waveguide for receiving light which can be used to cut hair. The invention also relates to a hair cutting device.

### BACKGROUND OF THE INVENTION

A shaving device has been proposed in WO 2014/143670 that makes use of laser light. In particular, a laser light source is provided that is configured to generate laser light having a wavelength selected to target a predetermined chromophore to effectively cut a hair shaft. An optical fibre is located on a shaving portion of the device that is positioned to receive the laser light from the laser light source at a proximal end, conduct the laser light from the proximal end toward a distal end, and emit the light out of a cutting region of the optical fibre and toward hair when the cutting region is brought in contact with the hair.

Over time, the optical fibre is heated and cooled repeatedly. The thermal and mechanical stresses experienced by the optical fibre may lead to the optical fibre becoming brittle and less stiff. The optical fibre may eventually break (i.e. become severed), and light propagating through the optical fibre may exit the optical waveguide via one or more severed faces created during the breakage.

Laser light coupling out from a severed face of the optical waveguide in an uncontrolled manner can be undesirable for a user, particularly if the laser light is directed towards the user's skin or eyes. In a known attempt to overcome the above-identified problem, it has been proposed to introduce a system by which, upon detecting that the optical fibre has been severed, the power to the laser light source is cut off, thereby preventing laser light from propagating through the optical fibre if it is broken. However, such a system introduces an additional step (i.e. detecting the breakage, then sending a signal to disable the laser source) and, therefore, there are more elements of system that could fail.

### SUMMARY OF THE INVENTION

Preventing laser light from being directed onto a user's skin or into a user's eyes will provide a better user experience, and will reduce the risk of temporary or even permanent damage from being caused to the user as a result of uncontrolled laser light coupling from a severed face of the optical waveguide.

Thus, there exists a need for an arrangement in which, in the event of an optical waveguide becoming severed, laser light is prevented from being directed towards a user's skin or eyes, or is blocked before the laser light is able to engage the user's skin or eyes.

According to a first aspect, there is provided a cutting assembly for use in a hair cutting device. The cutting assembly comprises an optical waveguide having a light receiving end and a sidewall, wherein a portion of the sidewall forms a cutting face for contacting hair. The cutting assembly further comprises a housing element to support the optical waveguide; and a biasing member acting upon the optical waveguide such that, upon the optical waveguide becoming severed, thereby creating two severed faces of the optical waveguide, the severed face attached to the light receiving end of the optical waveguide is caused to face the housing element.

By causing the optical waveguide to be urged in such a way that the severed face from which laser light may propagate is caused to face the housing element, laser light will not be able to couple out from the severed face into the user's skin or eyes. In this way, the user's hair cutting experience will be enhanced and the safety of the device is improved.

In some embodiments, the biasing member may be configured to tension the optical waveguide. In this way, tension in the optical waveguide will cause the optical waveguide to be urged into a desired position (i.e. with the severed face facing the housing element) in the event of the optical waveguide becoming severed.

The housing element may comprise a concave surface. The optical waveguide may be mounted on the concave surface. The biasing member may urge the optical waveguide towards the concave surface. By mounting the optical waveguide to a concave surface of the housing element, a severed face of the optical waveguide will naturally face the housing element in the event of the optical waveguide becoming severed.

In some embodiments, the housing element may comprise a channel for at least partially receiving the optical waveguide and securing the optical waveguide to the housing element. In this way, the optical waveguide can be mounted onto the housing element without the use of glue, which may be negatively affected by heat generated from the optical waveguide during use.

The housing element may comprise a plurality of mounting members for securing the optical waveguide to the housing element. By providing multiple mounting members, a severed face of the optical waveguide will be caused to face the housing element, wherever along the length of the optical waveguide the severing occurs.

In some embodiments, the housing element may support the optical waveguide at a first position and a second position. The biasing member may act upon the optical waveguide between the first position and the second position so as to urge the optical waveguide in a direction away from the cutting face. In use, the cutting face is typically positioned adjacent to the user's skin or hair. Therefore, by urging the optical waveguide away from the cutting face, a severed face of a severed optical waveguide will face away from the user's skin or hair.

The housing element may, in some embodiments, comprise a shield portion. The biasing member may act upon the optical waveguide such that, upon the optical waveguide becoming severed, the severed face attached to the first light receiving end is caused to face the shield portion. In this way, the shield portion may be positioned such that a severed face will be moved into a position in which laser light will be directed towards the shield portion, rather than towards the user's skin.

In some embodiments, the housing element may further comprise a guiding portion configured to guide the optical waveguide, upon the optical waveguide becoming severed, into a position in which the severed face attached to the first light receiving end is caused to face the shield portion. In some embodiments, multiple guiding portions may be provided.

The shield portion may be moveable between a first, retracted position and a second, engaged position. In the engaged position, the shield portion may be configured to shield the severed face of the optical waveguide.

In some embodiments, the cutting assembly may further comprise a sensor configured to detect a change in tension in the optical waveguide. The shield portion may be moved into the second, engaged position if the change in tension in the optical waveguide exceeds a defined threshold.

The biasing member may, in some embodiments, comprise a resilient member. The biasing member may comprise an extension spring.

The optical waveguide may have first and second light receiving ends. The biasing member may act upon the optical waveguide such that, upon the optical waveguide becoming severed, thereby creating two severed faces of the optical waveguide, the severed face attached to the first light receiving end and the severed face attached to the second light receiving end of the optical waveguide are caused to face the housing element.

In some embodiments, the optical waveguide may comprise an optical fibre.

According to a second aspect, there is provided a hair cutting device comprising a handle portion; a cutting assembly according to any of the preceding claims; and at least one light source connected to the light receiving end of the optical waveguide, for delivering light to the optical waveguide. A hair cutting device having a cutting assembly which exhibits the features discussed herein will provide a user with an enhanced and safer hair cutting experience.

Other advantageous features will become apparent from the following description.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a block diagram of a hair cutting device according to embodiments of the invention;
Fig. 2 is a pair of schematic drawings showing different views of an exemplary hair cutting device;
Fig. 3 is a schematic drawing of a cutting assembly according to embodiments of the invention;
Fig. 4 is a schematic drawing of an example of a cutting assembly according to embodiments of the invention;
Fig. 5 is a schematic drawing of a further example of a cutting assembly according to embodiments of the invention;
Fig. 6 is a schematic drawing of a further example of a cutting assembly according to embodiments of the invention;
Fig. 7 is a schematic drawing of a further example of a cutting assembly according to embodiments of the invention, with an un-severed optical waveguide (Fig. 7A) and with a severed optical waveguide (Fig. 7B); and
Fig. 8 is a schematic drawing of an example of a hair cutting device according to embodiments of the invention.

### DETAILED DESCRIPTION OF THE EMBODIMENTS

As noted above, the present invention provides an improvement in the safety and user satisfaction of a laser light-based shaving device, for example as described in WO 2014/143670. In particular, it has been recognized that, when an optical fibre in a laser light-based shaving device breaks, laser light may be directed from the severed faces of the optical fibre, towards the user's skin, or eyes. Such experience can be very unpleasant for a user, particularly if the power of the laser light is high enough to cause damage to the user. By designing the cutting assembly of the shaving device in such a way that light is unable to couple out from a severed face of the optical fibre into the skin or eyes of a user, the user can enjoy a safer and more enjoyable shaving experience.

It will be appreciated that the invention is applicable to shaving devices (e.g. razors or electric shavers), and any other type of device that is used to cut hair (e.g. hair clippers), even if those devices do not necessary aim to provide a 'clean shave' (i.e. to remove hair at the level of the skin).

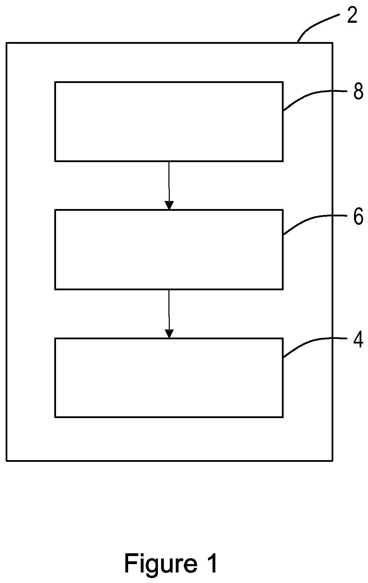

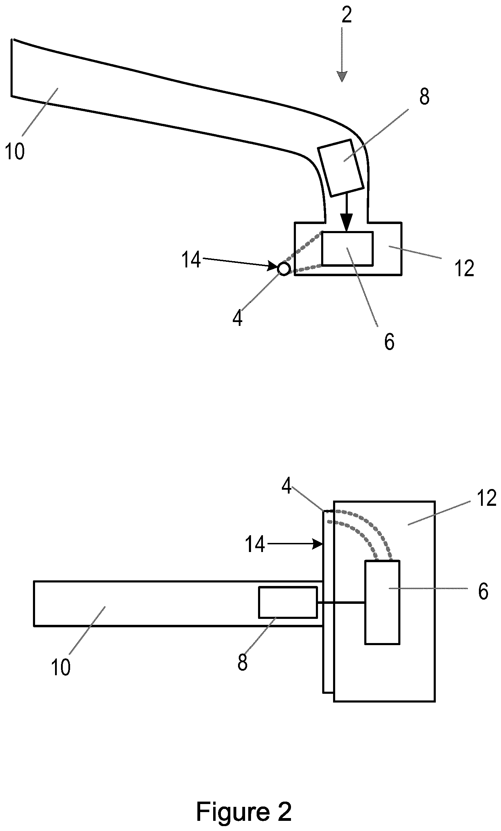

Fig. 1 is a block diagram of a hair cutting device 2 according to an embodiment of the invention. Fig. 2 shows a hair cutting device 2 in the form of a handheld razor according to an exemplary embodiment of the invention. The hair cutting device 2 is for cutting (e.g. shaving) hair on a body of a subject. The subject may be a person or an animal. The hair may be facial hair (i.e. hair on the subject's face), or hair on the subject's head or other part of their body (legs, chest, etc.).

The hair cutting device 2 comprises a cutting element 4 that enables hair to be cut as the hair cutting device 2 is moved over the skin of a subject. The cutting element 4 is an optical waveguide 4 that is arranged on the hair cutting device 2 so that the optical axis of the optical waveguide 4 (i.e. the line along which light typically propagates through the optical waveguide 4) is generally perpendicular to the direction in which the hair cutting device 2 is moved so that hairs contact the sidewall of the optical waveguide 4 (the sidewall corresponding to the long edge of the optical waveguide 4) as the hair cutting device 2 is moved across the skin of the subject. In some embodiments, the optical waveguide 4 is an optical fibre, although those skilled in the art will be aware of other types of optical waveguide that can be used according to the invention, such as a slab waveguide, a strip waveguide or a photonic crystal waveguide. An optical fibre comprises a core, and in some embodiments also comprises a cladding, which may or may not fully encompass the core (e.g. part of the core may be exposed). The optical waveguide 4 may form part of a cutting assembly of the hair cutting device 2. The cutting assembly may, in some embodiments, be a detachable and/or replaceable component, and may be designed to be replaced as the optical waveguide 4, or other components of the cutting assembly, become worn or damaged.

A light source 6 is provided in the hair cutting device 2 that generates laser light at one or more specific wavelengths. The light source 6 is optically coupled to the optical waveguide 4 so that the laser light generated by the light source 6 is coupled into the optical waveguide 4 (and specifically coupled into at least one end of the optical waveguide 4 so that the laser light propagates through the optical waveguide 4).

The light source 6 is configured to generate laser light at one or more specific wavelengths that can be used to cut or burn through hair. In particular, each wavelength corresponds to the wavelength of light absorbed by a chromophore that is found in hair. As is known, a chromophore is the part of a molecule that provides the molecule with its color. Thus, the laser light will be absorbed by the chromophore and converted into heat which will melt or burn the hair or otherwise destroy the bonds in the molecules of the hair, and it is this melting or burning that provides the cutting action of the hair cutting device 2.

Suitable chromophores that can be targeted by the laser light generated by the light source 6 include, but are not limited to, melanin, keratin and water. Suitable wavelengths of laser light that can be used include, but are not limited to, wavelengths selected from the range 380 nm (nanometres) to 500 nm and 2500 nm to 3500 nm. Those skilled in the art will be aware of the wavelengths of light that are absorbed by these chromophores, and thus also the specific wavelengths of light that the light source 6 should generate for this purpose, and further details are not provided herein.

In some embodiments the light source 6 can be configured to generate laser light at a plurality of wavelengths (either simultaneously or sequentially), with each wavelength being selected to target a different type of chromophore. This can improve the cutting action of the optical waveguide 4 since multiple types of molecules in the hair may be burnt using the laser light. Alternatively, multiple light sources 6 can be provided that each generate laser light at a respective wavelength. The multiple light sources may be coupled to a single optical waveguide, or each light source 6 can be coupled to a respective optical waveguide 4 to provide multiple cutting elements 4 in the device 2.

The hair cutting device 2 may also comprise a control unit 8 that controls the operation of the hair cutting device 2, and in particular may be connected to the light source 6 to control the activation and deactivation of the light source 6 (and in some embodiments control the wavelength and/or intensity of the light generated by the light source 6). The control unit 8 may activate and deactivate the light source 6 in response to an input from a user of the hair cutting device 2. The control unit 8 can comprise one or more processors, processing units, multi-core processors or modules that are configured or programmed to control the hair cutting device 2.

As noted above, Fig. 2 shows a hair cutting device 2 that is in the form of a handheld wet razor. Fig. 2 shows a side view and a bottom view of the razor 2. The razor 2 comprises a handle 10 for the subject (or other user of the device 2) to hold, and a head portion 12 that includes the cutting element 4 (optical waveguide/fibre). As shown, the optical waveguide 4 is arranged along an edge of the head portion, and a part of the optical waveguide 4 forms (or corresponds to) a cutting face 14. The cutting face 14 is the part of the optical waveguide 4 that is intended to come into contact with hair as the hair cutting device 2 is moved across the skin of the subject. A light source 6 and control unit 8 are shown as being incorporated into the head portion 12 and handle 10 respectively, but it will be appreciated that the positions of these components in the hair cutting device 2 as shown in Fig. 2 is not limiting. Likewise, it will be appreciated that the embodiment shown in Fig. 2 is merely an example, and the invention can be incorporated or used in any type of hair cutting device 2 that comprises an optical waveguide cutting element 4 as described herein.

As is known, the optical waveguide 4 acts as a waveguide for the light coupled from the light source 6 through the occurrence of total internal reflection, since the refractive index of air is lower than that of the optical waveguide 4. However, if an object that has a refractive index higher than the optical waveguide 4 is put into contact with the optical waveguide 4, then the total internal reflection is 'frustrated' and light can couple from the optical waveguide 4 into that object. Thus, in order for light to be coupled into a hair from the optical waveguide 4 (to provide the cutting action according to the invention), the optical waveguide 4 should preferably have the same or a lower refractive index than hair at the point at which the hair contacts the optical waveguide 4. Thus, the optical waveguide 4 should preferably have the same or a lower refractive index than hair at least at the cutting face 14 portion of the optical waveguide 4. Preferably the refractive index of the optical waveguide 4 at the cutting face 14 is the same as that of hair since that provides the best coupling of light from the optical waveguide 4 to the hair. Light may still be able to couple from the optical waveguide 4 into an object (e.g. a hair) brought into contact with the cutting face 14 of the optical waveguide even if the refractive index of the optical waveguide is higher than that of the object, due to a high numerical aperture in the cutting face.

The hair cutting device (and components thereof) described herein has been designed specifically to ensure that, in the event that the optical waveguide becomes severed due to wear and tear, due to the waveguide becoming brittle as a result of repeated temperature increases and decreases, due to sudden damage (e.g. a sudden impact), or due to some other cause, the severed faces of the optical waveguide do not face the user, or are at least shielded from the user. As used herein, the term "severed face" refers to a cross-sectional face of an optical waveguide which is formed as a result of the optical waveguide being severed.

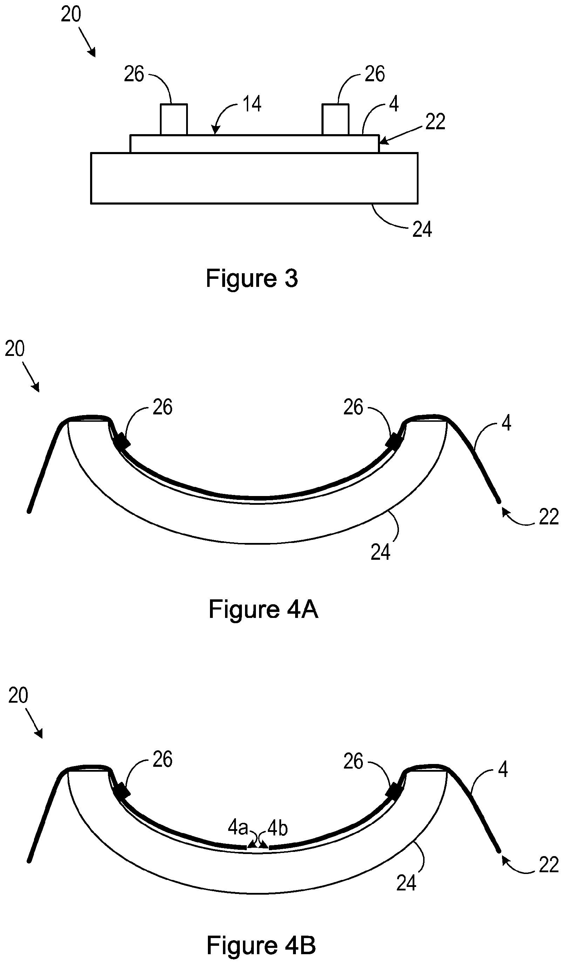

Fig. 3 is a schematic drawing of an example of a cutting assembly 20 for use in a hair cutting device. The cutting assembly 20 comprises an optical waveguide 4 having a light receiving end 22 and a sidewall 14, wherein a portion of the sidewall forms a cutting face for contacting hair. The cutting assembly 20 further comprises a housing element 24 to support the optical waveguide 4. The cutting assembly 20 further comprises a biasing member 26 acting upon the optical waveguide 4 such that, upon the optical waveguide becoming severed, thereby creating two severed faces of the optical waveguide, the severed face attached to the light receiving end 22 of the optical waveguide is caused to face the housing element 24. The embodiment of Fig. 3 includes two biasing members 26, but it will be appreciated that, in other embodiments, the cutting assembly may include a single biasing member, or more than two biasing members.

The design of a feature or component to ensure it is safe in the event of that feature or component failing is known as safety-by-design. The cutting assembly 20 as described herein is considered to fall within the safety-by-design category as the features of the cutting assembly are such that any additional action (beyond the optical waveguide becoming severed) takes place automatically to ensure that the severed face is prevented from directing laser light into the user's eyes or onto the user's skin.

The invention, the general features of which are shown in Fig. 3, will be described in terms of numerous different embodiments, as shown in Figs. 4 to 8. In the embodiments, the biasing member 26 may be configured to tension the optical waveguide.

Fig. 4 is a schematic drawing of an example of a cutting assembly 20 according to embodiments of the invention. Fig. 4A shows the cutting assembly 20 having an un-severed optical waveguide 4 (i.e. an optical waveguide in its normal, functioning configuration) and Fig. 4B shows the cutting assembly 20 after the optical waveguide has been severed. Referring to Fig. 4A, the cutting assembly 20 includes the optical waveguide 4, the housing element 24 and the biasing member 26. In this example, the housing element 24 comprises a concave surface. The optical waveguide 4 is mounted on the concave surface, and the biasing member 26 urges the optical waveguide towards the concave surface. In this embodiment, the biasing member 26 comprises a member positioned near to each end of the housing element 24. Each biasing member 26, which may comprise a clip or a support, urges the optical waveguide towards the housing element 24. The ends of the optical waveguide 4 may be attached to another portion of the hair cutting device 2 or to another portion of the housing element 24. At least one end of the optical waveguide 4 comprises a light receiving end 22, and may be coupled to a laser light source (not shown). In some embodiments, the cutting assembly 20 may comprise additional biasing members 26, used to mount or secure the optical waveguide 4 to the housing element 24, spaced along the optical waveguide 4. Thus, in some embodiments, the housing element 24 may comprise a plurality of mounting members for securing the optical waveguide 4 to the housing element.

In this embodiment, the biasing members 26 serve to tension the optical waveguide 4 by urging it towards the concave surface of the housing element 24, and the concave shape of the housing element is used to ensure that a severed face of the optical waveguide 4 remains facing the housing element (rather than facing the user directly) after a severing event. Fig. 4B shows the arrangement of Fig. 4A after the optical waveguide 4 has been severed. The severing of the optical waveguide 4 causes the creation of two severed faces 4a and 4b. The biasing members 26 continue to urge the optical waveguide 4 towards the housing element 24, so the severed ends 4a and 4b remain against the housing element, facing the housing element. In this way, if light were to be directed out of, or couple out from, the optical waveguide via one of the severed faces 4a, 4b, then it would be directed at the housing element, not at the user.

In some embodiments, the housing element 24 may comprise a channel or groove (not shown) for at least partially receiving the optical waveguide 4 and securing the optical waveguide to the housing element. In this way, the optical waveguide 4 may remain attached to the housing element 24 after the optical waveguide has been severed, such that the severed faces remain facing the housing element, and are not able to become detached such that they could face the user's skin. In some embodiments, the housing element 24 may comprise a flat surface upon which the optical waveguide is mounted. Tension in the optical waveguide 4 may be created by biasing members 26, such that, in the event that the optical waveguide were to be severed, the severed ends would remain against the housing element 24, such that the severed faces remain directed at the housing element.

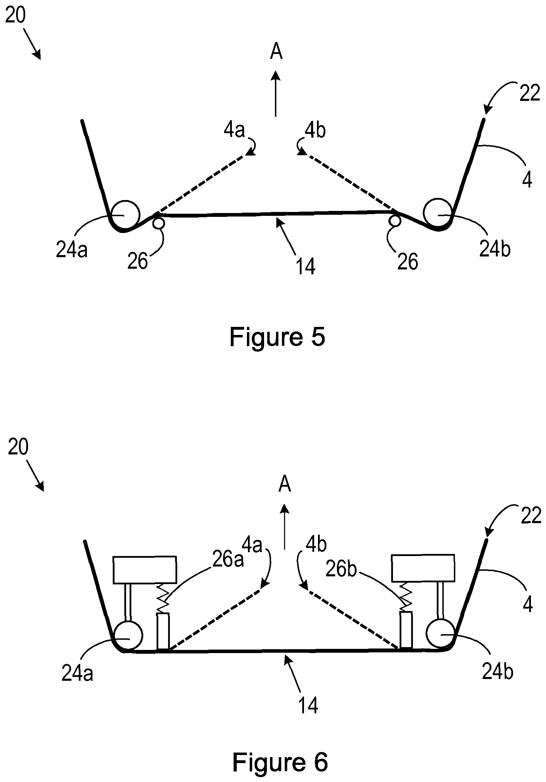

In the embodiment shown in Figs. 4A and 4B, the shape of the housing element 24 upon which the optical waveguide 4 is mounted serves to secure the severed ends 4a, 4b in such a manner that they face the housing element after the optical waveguide has been severed. In other embodiments, additional structures may be provided, or portions of the housing element may be arranged to cause the optical waveguide to move into a desired defined position after it has been severed. Figs. 5 and 6 shown examples of cutting assemblies in which the optical waveguide 4 is urged into a particular position after a severing event.

In the embodiment shown in Fig. 5, a portion of the housing element 24 supports the optical waveguide 4 at a first position 24a and a second position 24b. As explained in relation to the embodiment of Fig. 4, the optical waveguide 4 may be connected at each end to the housing element 24 or to another part of the hair cutting device 2. For example, the light receiving end 22 may be coupled to a laser light source (not shown). At the positions 24a and 24b, at which the housing element 24 supports the optical waveguide 4 in this embodiment, the housing element may comprise rollers, notches or other structures capable of supporting the optical waveguide in a particular position relative to the hair cutting device 2. The biasing member 26 acts upon the optical waveguide between the first position 24a and the second position 24b so as to urge the optical waveguide 4 in a direction away from the cutting face 14. In this embodiment, the biasing member 26 comprises a pair of notches, lugs or the like which urge the optical waveguide in a direction shown by the arrow A. The biasing members 26 in conjunction with the housing element 24 serve to tension the optical waveguide 4. In the event that the optical waveguide breaks, the force of the biasing members 26 acting on the optical waveguide 4 would urge the severed ends of the optical waveguide in the direction A, into a configuration indicated by the dashed lines. With the optical waveguide 4 being urged in this way, the severed faces 4a, 4b of the optical waveguide face a portion of the housing element 24, such that any laser light escaping the optical waveguide through the severed ends is not directed at the user's skin or eyes. It is noted that the optical waveguide 4 is typically stiff enough, and short enough (i.e. the length of the cutting face may be between around 2 centimetres and 4 centimetres long) that, once severed, the severed ends of the optical waveguide will remain relatively straight, and will not bend or fold.

Fig. 6 shows an embodiment similar to that shown in Fig. 5. In Fig. 6, the biasing member 26 comprises a pair of extension springs 26a, 26b, which are connected to the optical waveguide 4. The extension springs 26a, 26b urge the optical waveguide in the direction A, thereby tensioning the optical waveguide 4. In the event of the optical waveguide 4 becoming severed, extension springs 26a, 26b resume their un-extended configuration, causing the severed ends to move into the position shown by the dashed lines, such that the severed faces 4a, 4b of the optical waveguide face a portion of the housing element 24.

In any of the embodiments disclosed herein, the biasing member may comprise a resilient member. The biasing member may, in some embodiments, such as the embodiment described above, comprises an extension spring.

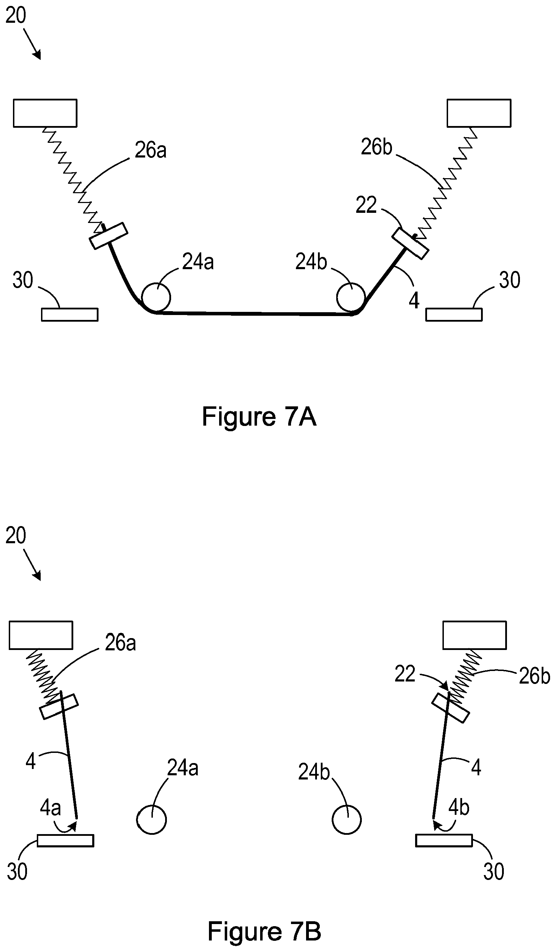

Figs. 7A and 7B show an embodiment in which severed ends of the optical waveguide are moved into a position in which the severed faces will be shielded, such that laser light escaping from the severed faces is not directed on a user's skin or into the user's eyes. The optical waveguide 4 is supported at first and second positions 24a, 24b by the housing element 24.

In the embodiment shown in Fig. 7A, the optical waveguide 4 is attached at its ends to biasing member 26a, 26b which, in this embodiment, comprise extension springs. The extension springs tension the optical waveguide 4. The light receiving end 22 of the optical waveguide 4 may be coupled to a light source (not shown). In this embodiment, a portion of the housing element 24 comprises a shield portion 30. If the optical waveguide 4 is severed, the extension springs 26a, 26b resume their un-extended configuration, causing the severed ends to move into the position shown in Fig. 7B. The severed ends of the optical waveguide 4 are retracted into a position in which the severed faces 4a and 4b face the shield portions 30 of the housing element 24. Thus, the biasing member 26 acts upon the optical waveguide 4 such that, upon the optical waveguide becoming severed, the severed face 4a, 4b attached to the first light receiving end 22 is caused to face the shield portion 30.

It will be apparent that, following a severing event, the portions of the housing element 24 at positions 24a and 24b will, to some extent, serve to guide the optical waveguide 4 into the position shown in Fig. 7B. In some embodiments, the housing element 24 may further comprise a guiding portion (not shown) configured to guide the optical waveguide 4, upon the optical waveguide becoming severed, into a position in which the severed face 4a, 4b attached to the first light receiving end 22 is caused to face the shield portion 30.

According to some embodiments, the shield portion 30 be moveable between a first, retracted position and a second, engaged position. Movement of the shield portion of the housing element 24 maybe effected, for example, using a mechanical or electrical actuation means. In the engaged position, the shield portion 30 may be configured to shield the severed face 4a, 4b of the optical waveguide 4. In this way, the shield portion 30 may be positioned such that it does not interfere with the optical waveguide 4 while the cutting assembly 20 is in use. However, the shield portion 30 can be brought into the intended position (e.g. the position shown in Fig. 7B) when a severing event is detected, such that the severed face 4a, 4b of the optical waveguide is rapidly shielded from the user.

In embodiments in which the shield portion 30 is moveable between a retracted position and an engaged position, the cutting assembly may further comprise a sensor (not shown) configured to detect a change in tension in the optical waveguide 4. The sensor may, for example, be a mechanical, electrical or optical sensor. The shield portion 30 may be moved into the second, engaged position if the change in tension in the optical waveguide 4 exceeds a defined threshold. A defined threshold is set such that small changes in the tension, resulting, for example, from normal use of the cutting assembly, do not trigger the movement of the shield portion 30 into the engaged position.

In the embodiments discussed herein, the optical waveguide 4 has been described as having a single light receiving end 22, which may be coupled to a light source. However, as discussed above, in some embodiments, multiple light sources may be provided, and laser light may be coupled into both ends of the optical waveguide. In such embodiments, the optical waveguide 4 may have first and second light receiving ends. The biasing member 26 may act upon the optical waveguide such that, upon the optical waveguide 4 becoming severed, thereby creating two severed faces of the optical waveguide, the severed face attached to the first light receiving end and the severed face attached to the second light receiving end of the optical waveguide are caused to face the housing element 24. Thus, any severed face 4a, 4b from which light may escape is moved into a position such that the light is directed at a portion of the housing element, to avoid the light encountering the user.

It will be appreciated that the optical waveguide 4 may, in some embodiments, comprise an optical fibre. In other embodiments, the optical waveguide 4 may comprise some other form of waveguide suitable for receiving laser light, and suitable for coupling laser light out from a cutting face into a user's hair.

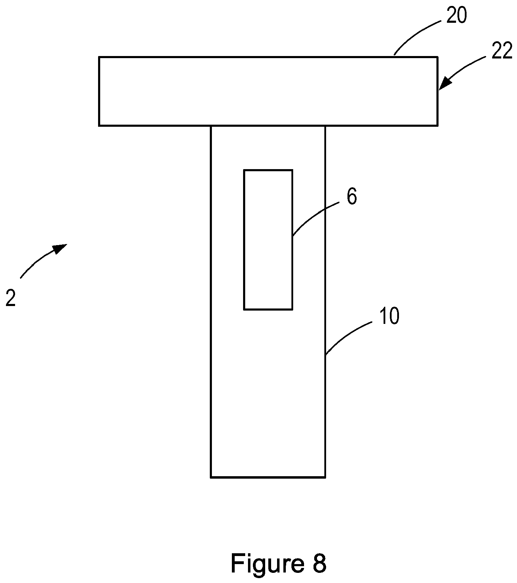

The cutting assemblies described above may be incorporated into various shaving or hair cutting devices. One such hair cutting device 2 is shown schematically in Fig. 8. The hair cutting device comprises a handle portion 10, a cutting assembly 20 as described herein, and at least one light source 6 connected to the light receiving end 22 of the optical waveguide, for delivering light to the optical waveguide.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. Any reference signs in the claims should not be construed as limiting the scope.

## Claims

1. A cutting assembly for use in a hair cutting device, the cutting assembly comprising:
an optical waveguide having a light receiving end and a sidewall, wherein a portion of the sidewall forms a cutting face for contacting hair;
a housing element to support the optical waveguide; and
a biasing member acting upon the optical waveguide such that, upon the optical waveguide becoming severed, thereby creating two severed faces of the optical waveguide, the severed face attached to the light receiving end of the optical waveguide is caused to face the housing element.

2. A cutting assembly according to claim 1, wherein the biasing member is configured to tension the optical waveguide.

3. A cutting assembly according to claim 1 or claim 2, wherein the housing element comprises a concave surface;
wherein the optical waveguide is mounted on the concave surface; and
wherein the biasing member urges the optical waveguide towards the concave surface.

4. A cutting assembly according to any of the preceding claims, wherein the housing element comprises a channel for at least partially receiving the optical waveguide and securing the optical waveguide to the housing element.

5. A cutting assembly according to any of the preceding claims, wherein the housing element comprises a plurality of mounting members for securing the optical waveguide to the housing element.

6. A cutting assembly according to claim 1 or claim 2, wherein the housing element supports the optical waveguide at a first position and a second position; and
wherein the biasing member acts upon the optical waveguide between the first position and the second position so as to urge the optical waveguide in a direction away from the cutting face.

7. A cutting assembly according to claim 1 or claim 2, wherein the housing element comprises a shield portion; and
wherein the biasing member acts upon the optical waveguide such that, upon the optical waveguide becoming severed, the severed face attached to the first light receiving end is caused to face the shield portion.

8. A cutting assembly according to claim 7, wherein the housing element further comprises a guiding portion configured to guide the optical waveguide, upon the optical waveguide becoming severed, into a position in which the severed face attached to the first light receiving end is caused to face the shield portion.

9. A cutting assembly according to claim 7 or claim 8, wherein the shield portion is moveable between a first, retracted position and a second, engaged position;
wherein, in the engaged position, the shield portion is configured to shield the severed face of the optical waveguide.

10. A cutting assembly according to claim 9, further comprising a sensor configured to detect a change in tension in the optical waveguide;
wherein the shield portion is moved into the second, engaged position if the change in tension in the optical waveguide exceeds a defined threshold.

11. A cutting assembly according to claim 6 or claim 10, wherein the biasing member comprises a resilient member.

12. A cutting assembly according to any of claims 6 to 11, wherein the biasing member comprises an extension spring.

13. A cutting assembly according to any of the preceding claims, wherein the optical waveguide has first and second light receiving ends; and
wherein the biasing member acts upon the optical waveguide such that, upon the optical waveguide becoming severed, thereby creating two severed faces of the optical waveguide, the severed face attached to the first light receiving end and the severed face attached to the second light receiving end of the optical waveguide are caused to face the housing element.

14. A cutting assembly according to any of the preceding claims, wherein the optical waveguide comprises an optical fibre.

15. A hair cutting device comprising:
a handle portion;
a cutting assembly according to any of the preceding claims; and
at least one light source connected to the light receiving end of the optical waveguide, for delivering light to the optical waveguide.
